# EUROPEAN PATENT APPLICATION

(11) **EP 3 085 766 A1**
(43) Date of publication of application: **26.10.2016**
(21) Application number: 16165589.9
(22) Date of filing: 15.04.2016
(51) Int. Cl.: C12M 1/107, C12M 1/06, C12M 1/26

(54) **SYSTEM FOR PRODUCING AND DISTRIBUTING BIOGAS**

(30) Priority: 17.04.2015 FI 20155286
(71) Applicant: Biogts OY, 40320 Jyväskylä (FI)
(72) Inventor: LEHTOMÄKI, Annimari, 41900 PETÄJÄVESI (FI); RAUTIAINEN, Mika, 41310 LEPPÄVESI (FI)
(74) Representative: Helke, Kimmo Kalervo

(57) **Abstract**

The invention is related to a system for producing and distributing biogas, the system (10) including
- a biogas reactor (12) for producing biogas from biomaterial with dry anaerobic digestion,
- first conveyor units (20) for transporting biomaterial,
- first handling equipment (18) for unloading biomaterial,
- first transfer equipment (19) for supplying biomaterial to the biogas reactor (12)
- second transfer equipment (22) for unloading digestate from the biogas reactor (12),
- second conveyor units (21) for carrying away digestate,
- biogas recovery equipment (28), a biogas upgrading unit (32), a depot (30) and a refueling unit (34) for distributing biogas from the depot (30) to a user.

The first conveyor units (20) and the second conveyor units (21) are transfer containers (36) adapted to be transported by lorries (48), and the system (10) further includes at least one lorry (48) for transporting said transfer containers (36).

## Description

The present invention relates to a system for producing and distributing biogas, the system including
- a biogas reactor having a first end and a second end for producing biogas from biomaterial with dry anaerobic digestion,
- first conveyor units for conveying biomaterial functioning as the raw material to the biogas reactor,
- first handling equipment for unloading biomaterial from the first conveyor unit,
- first transfer equipment arranged at the first end of the biogas reactor for supplying biomaterial to the first end of the biogas reactor,
- second transfer equipment set at the second end of the biogas reactor for unloading digestate produced from biomaterial from the second end of the biogas reactor,
- second conveyor units for carrying digestate away from the biogas reactor,
- biogas recovery equipment located in association with the biogas reactor for recovering biogas produced in the biogas reactor during the dry anaerobic digestion of biomaterial,
- a biogas upgrading unit connected to the biogas recovery equipment for purifying biogas,
- a depot for storing purified biogas obtained from the upgrading unit, and
- a refueling unit for distributing biogas from the depot to a user.

An EU target is to promote the availability of gaseous traffic fuels (natural gas and biomethane) to realise a comprehensive network of gas refueling stations spanning the entire area of the European union by 2020 with a maximum distance of 150 km between the refueling points of gaseous fuels. These EU targets are a strong driver for the development of a gas refueling station network that is resting upon biomethane and is external to the natural gas network. In Finland, the realisation of these targets would practically mean the development of a refueling station network for gaseous fuels consisting of approximately 650 refueling points by 2020.

Today, gaseous fuels or biogases are produced in separate production plants from which biogas is transported to the refueling locations of refueling networks. The production plants are often large complexes, such as centralised waste treatment plants, farms or landfill sites, where biogas is principally generated as a result of digestion. One such production chain is described in the presentation "Biokaasua maatilalta" (Biogas from a farm) that was given by Erkki Kalmari from the Finnish Metener Oy on 20 February 2014 in the Vocational College Lappia. Today, only a few production plants exist and they are distantly located and thus, biomaterial for raw material use must be transported long distances. Similarly, the transport distance of upgraded biogas from the production plant to refueling stations is often quite long. On the other hand, operation of large production plants requires work force, which is expensive.

A system for producing biogas from biowaste is known from publication WO 2014/210071 A1. The system includes a biogas reactor and a upgrading unit, to which biowaste functioning as the raw material is conveyed in individual waste containers. However, the use of waste containers for transporting biowaste is inefficient and requires personnel to place the waste containers in handling equipment that dumps the contents of the waste container and supplies the biowaste to the biogas reactor.

The object of the invention is to provide a system that is better than prior art systems for producing and distributing biogas, by which system biogas can be produced in association with a distribution point with a remarkably affordable system that can be automated. The characteristic features of this invention are set forth in the appended claim 1.

This object can be achieved with a system for producing and distributing biogas, said system including a biogas reactor having a first end and a second end for producing biogas from biomaterial with dry anaerobic digestion, first conveyor units for transporting biogas functioning as the raw material to the biogas reactor, and second conveyor units for transporting digestate from the biogas reactor. The first conveyor units and the second conveyor units are transfer containers adapted to be transported by lorries, and the system further includes at least one lorry for transporting transfer containers. In addition, the system includes first handling equipment for unloading biomaterial from the first conveyor unit, first transfer equipment arranged at the first end of the biogas reactor for supplying biomaterial to the first end of the biogas reactor and second transfer equipment set at the second end of the biogas reactor for unloading digestate produced from biomaterial from the second end of the biogas reactor. The system also includes biogas recovery equipment located in association with the biogas reactor for recovering biogas produced in the biogas reactor during the dry anaerobic digestion of biomaterial, a biogas upgrading unit connected to the biogas recovery equipment for purifying biogas, a depot for storing purified biogas obtained from the upgrading unit, and a refueling unit for distributing biogas from the depot to a user.

In a system according to the invention, normal lorries can be used to efficiently transport large amounts of biowaste in transfer containers adapted to be transported by lorries. A transfer container is sufficiently large as a conveyor unit to obtain abundantly raw material from one transfer container during one unloading. The unloading and filling of a transfer container can be arranged fully automated by automating the first handling equipment, in which case the entire system can be operated under remote control. Thus, the system can even be implemented without onsite personnel or at least with an extremely reduced need of workforce. In addition, a closed transfer container prevents odours or liquid of biowaste or digestate from spreading into the environment thus allowing the biogas reactor to be located closer to residential areas. In this context, transfer containers mean transfer containers that are suitable for road traffic and can be transported using a normal lorry.

The biogas reactor may be an elongated channel-like reactor with two ends, to the first of which biowaste functioning as the raw material is supplied and from the second of which digestate is discharged. The use of transfer containers is particularly advantageous in association with an elongated biogas reactor, since transfer containers can be functionally connected to the ends of the biogas reactor.

Preferably, the biogas reactor is a continuously operating plug-flow reactor. A continuously operating reactor is easier to automate than a batch reactor due to a continuous and uniform raw material supply and thereby consistent gas production and thus, it is better suited to the application according to the invention. In association with a plug-flow reactor, the use of transfer containers is particularly advantageous, since transfer containers can be functionally connected to a biogas reactor having two ends.

Preferably, the biogas reactor is an elongated channel-like biogas reactor. Thus, the supply to its one end and the discharge from its other end can be implemented using transfer containers.

Preferably, the first conveyor units and the second conveyor units are alike. Thus, the same conveyor units can be used for both the transport of biowaste and the discharge of digestate. In this way, the manufacture of conveyor units of a system according to the invention becomes easier and is economically more affordable when all conveyor units can be alike.

According to an embodiment, the first handling equipment has been formed in each first conveyor unit. Using the handling equipment included in each first conveyor unit, the first conveyor unit can be efficiently unloaded and biomaterial will not be stuck within the transfer container. In other words, the first handling equipment is located in the transfer container.

According to an embodiment, equipment is arranged in the system for the first handling equipment formed in the transfer containers for supplying driving force to the first handling equipment.

According to an embodiment, the first handling equipment is a bottom conveyor fitted within the transfer container that functions as the first conveyor unit, the bottom conveyor preferably being a pull bar unloader. With the bottom conveyor, each first transfer unit can be efficiently unloaded and the unloading can be automated.

According to a second embodiment, the first handling equipment is a push wall fitted within the transfer container that functions as the first conveyor unit. A push wall is an industrial construction that is very commonly used for unloading transfer containers and it is quite affordable regarding its investment costs. In addition, a push wall unloads a transfer container reliably and empties it fully.

According to a third embodiment, the first handling equipment is bottom unloading equipment fitted in the transfer container. The unloading of a transfer container with a bottom unloading system is very easy to automate.

The first handling equipment can be tipping equipment for tipping the first conveyor unit. With the tipping equipment, all of the first conveyor units can be unloaded using the same equipment, in which case the first conveyor unit can be manufactured without separate in-built transfer equipment. During tipping, biowaste flows out of the transfer container by the force of gravity.

The system may include container handling equipment for moving transfer containers to and from a lorry. In this way, the handling of transfer containers is quick and convenient.

Preferably, container handling equipment can be the loading equipment for transfer pallets associated with each lorry. Most lorries are generally equipped with loading equipment.

Alternatively, container handling equipment may be associated with the biogas reactor. In this case, container handling equipment is not needed in each lorry.

As regards the system components, at least the biogas reactor and the upgrading unit can be arranged within an element or elements, which enables easy implementation of the system and relatively low investment costs compared to prior art. The design of the biogas reactor enables the manufacture of easily scalable biogas reactors with low investment costs near the source of biomaterial used as the raw material and, at the same time, near traffic connections. The use of a transfer container or preferably transfer containers for transferring biomaterial and digestate enables a simple material transfer using general transportation equipment. At the same time, a transfer container is a sealed construction preventing spreading of any raw material odours or liquids into the environment during transportation.

Preferably, the biogas reactor is a continuously operating plug-flow reactor formed of at least two modular reactor blocks. Regarding its construction, a modular biogas reactor is easily scalable to different size classes, since the volume of the reactor can be increased or reduced by changing the number of reactor blocks or reactors.

The system preferably includes a pump for pressurising biogas discharged from the biogas reactor to 5 - 20 bar for the upgrading unit. Thus, biogas is at a suitable pressure for further upgrading.

According to an embodiment, following the second transfer equipment, the plant may also be provided with digestate separation equipment, which divides digestate into a solid fraction and a liquid fraction, in which case both have a conveyor unit of their own.

The depot may be adapted to store biogas at a pressure of 200 - 300 bar. A high storage pressure enables biogas to be discharged from the depot without using a compressor, in which case biogas will not be heated during pumping. In this way, biogas can be refueled colder than in prior art refueling units, which enables a larger biogas volume to be fitted in a user's vehicle tank and a faster refueling operation.

Preferably, the system may also include a gas boiler for meeting the system's own heating demand using the biogas produced. Thus, the operation of the system is self-sufficient in terms of energy and relatively independent of external factors. Alternatively, the system may also be connected to an external heat network, if heat is affordably available.

Preferably, both the first transfer equipment and the second transfer equipment include vacuum generation equipment for generating a vacuum in the transfer container to prevent odour emissions. Due to the efficient prevention of odour emissions, the system can also be located close to residential areas, where a biogas distribution network is mostly needed. Odour gases generating from the raw material can be treated with ozonation.

Preferably, the refueling unit is a refueling unit for biomethane upgraded for road traffic purposes. Thus, the system can be used to produce and distribute biomethane directly to an end user from the same place in which the production takes place.

According to an embodiment, all of the units of the system are located within elements. This increases the simplicity of system transfers and its installation.

Preferably, the system includes second handling equipment for filling transfer containers with digestate discharging from the biogas reactor.

Preferably, the system includes several lorries for transporting transfer containers. Thus, raw material can be efficiently obtained for the biogas reactor.

Preferably, the first transfer equipment and the second transfer equipment are also adapted to be placed within / in association with the same element of the biogas reactor. These are included in the system according to the invention as one of its important component and are therefore preferably located in the same element.

Preferably, the transfer container is a closed construction that helps prevent odours from spreading into the environment from the transfer container.

Preferably, the first handling equipment is arranged to completely unload the transfer container. In this way, the transport capacity of the transfer container can be utilised for transportation. In this context, complete unloading of the transfer container means that at least 99 % of the contents of the transfer container is unloaded with the first handling equipment.

According to an embodiment, the volume of the transfer container is at least 20 m³. Thus, transportation is efficient, since a large amount of biowaste can be transferred with one transfer trip.

According to an embodiment, the dimensions of the transfer container are: height 2.5 m, width 2.0 m and length 6.0 m.

The first handling equipment may be adapted to unload the transfer container slowly according to the usage of biowaste in the biogas reactor so that the unloading takes 48 hours at a minimum, if the system is implemented without an intermediate depot. Thus, the transfer container also functions as an intermediate depot for both the biowaste supplied and the digestate discharged. Naturally, the lorry then leaves the transfer container in the vicinity of the first handling equipment for the duration of the feeding operation.

Alternatively, when using an intermediate depot, transfer containers may be unloaded fast, in a few minutes.

According to an embodiment, the system includes automation equipment for automating the system functions. In practice, this means that the supply of biowaste to the biogas reactor is automated, i.e. the lorry driver leaves the transfer container on the platform, turns on the automation equipment and delivers information to the automation equipment about the connection of a new container. The supply from the transfer container to the first transfer equipment and further to the biogas reactor takes places automatically according to the need of a new input of the biogas reactor.

The automation equipment preferably includes mechanical coupling equipment for connecting the transfer container to the system, said coupling equipment keeping the transfer container in place. Automation equipment controls the first transfer equipment for feeding biowaste to the biogas reactor preferably based on the biological loading status of the biogas reactor and the demand of gas.

Automation equipment may include a computer and software means to automatically control the system actuators based on measurements.

In a system according to the invention, the idea is to use lorries and transfer containers that are generally used in lorries for moving biowaste to a biogas reactor, which can be located near residential areas and heavy traffic routes. On the other hand, a system that is preferably formed of elements is easily scalable by connecting biogas reactors in parallel to generate the necessary capacity. Furthermore, the system being preferably assembled from elements, the need of a separate building for a biogas reactor is eliminated. As transfer containers for unloading raw material and waste, it is possible to use normal transfer containers for traffic purposes, which can be transported with normal lorries equipped with systems for loading transport platforms. Furthermore, transfer containers keep odour gases generating in biomaterial applied as the raw material tightly inside preventing odour impacts to the environment.

The invention is described below in detail by referring to the appended drawings, which illustrate some of the embodiments of the invention, in which:
- Figure 1: is an axonometric basic view of a system according to the invention,
- Figure 2: is a basic layout view of a system according to the invention,
- Figure 3: is a more detailed view of the construction of the biogas reactor of a system according to the invention,
- Figure 4a: is a lateral sectional view of a portion of a transfer container of a system according to the invention, the transfer container including a pull bar unloader as the first handling equipment for unloading biowaste from the transfer container,
- Figure 4b: is a lateral sectional view of a portion of a transfer container of a system according to the invention, the transfer container including an unloader mat as the first handling equipment for unloading biowaste from the transfer container,
- Figure 4c: is a lateral sectional view of a portion of a transfer container of a system according to the invention, the system including tipping equipment integrated into the a lorry as the first handling equipment for unloading biowaste from the transfer container,
- Figure 4d: is a lateral sectional view of a portion of a transfer container of a system according to the invention, the system including tipping equipment separate from the lorry as the first handling equipment for unloading biowaste from a transfer container loaded on the lorry,
- Figure 4e: is a lateral sectional view of a portion of a transfer container of a system according to the invention, the system including tipping equipment separate from the lorry as the first handling equipment for unloading biowaste from the transfer container,
- Figure 5a: is a lateral sectional view of a portion of a transfer container of a system according to the invention, the transfer container including a push wall as the first handling equipment for unloading biowaste from the transfer container,
- Figure 5b: is a rear view of the transfer container of figure 5a,
- Figure 5c: is a front view of the transfer container of figure 5a.

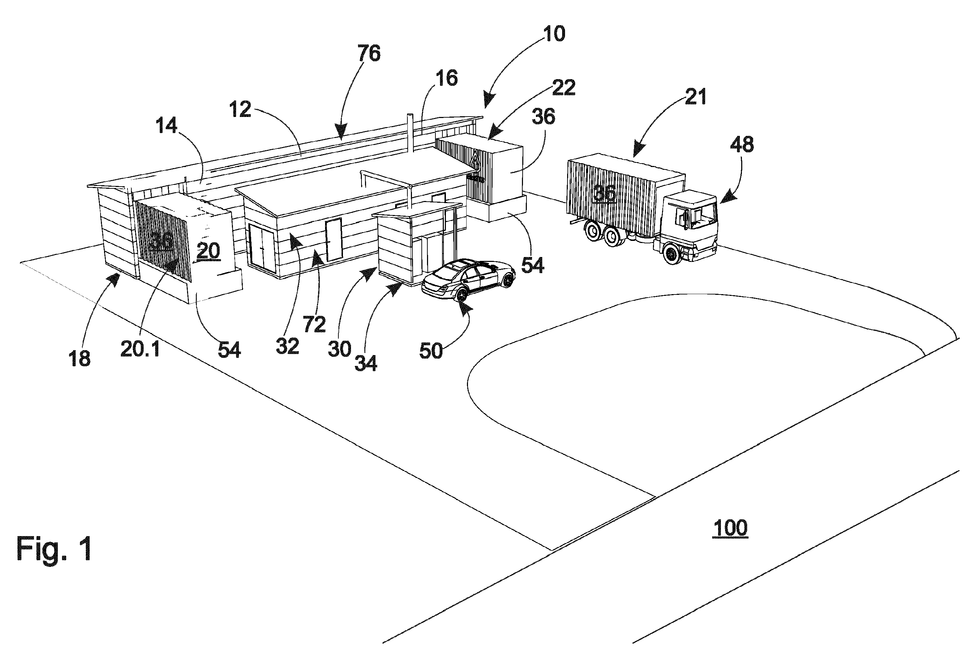

Figure 1 illustrates an embodiment of a system according to the invention, where the system 10 for producing and distributing biogas is formed in the vicinity of a road traffic connection 100. The system 10 forms an entity into which biomaterial used as the raw material can be brought with at least one transfer container 36 using lorries 48 and from which an end user 50 can finally buy biogas. In the embodiment of figure 1, the system is adapted for the production and distribution of biomethane suited to road traffic. Preferably, the system is formed near road traffic connections 100, preferably along a heavily trafficked road 100 in an area where raw material is available in nearby regions. Raw material may be, for example, agricultural waste, municipal waste or other equivalent biomaterial that can be used to produce biogas by dry anaerobic digestion.

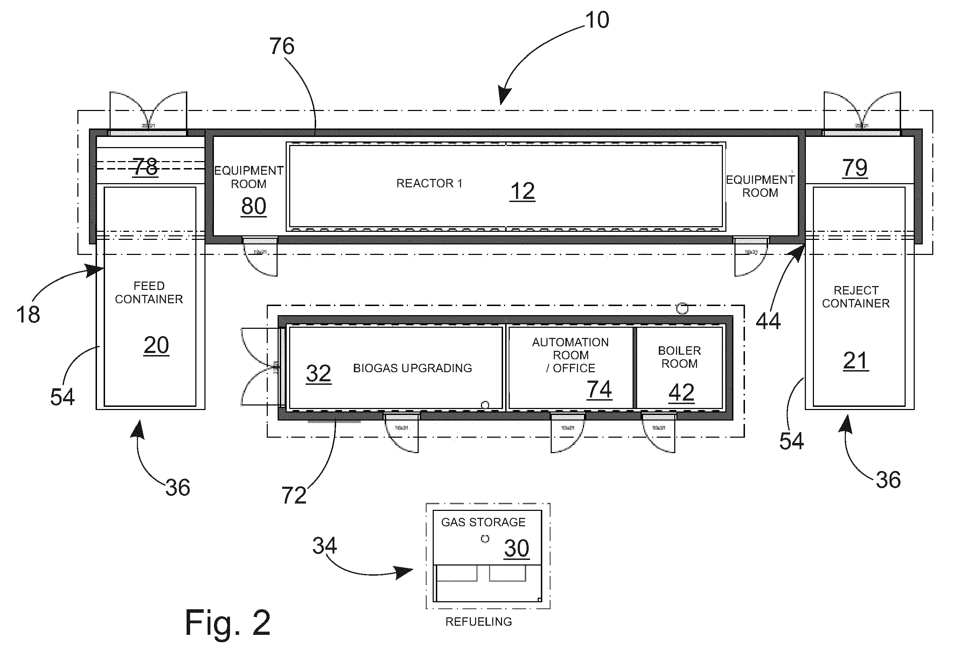

According to figure 2, the system 10 according to the invention includes at least a biogas reactor and a upgrading unit, which are preferably fitted inside prefabricated elements. In this context, a prefabricated element means a construction prefabricated in a factory, within which a biogas reactor and/or a upgrading unit are fitted. Preferably, the system includes as units a biogas reactor 12 of the plug-flow reactor type formed of a minimum of two modular reactor blocks 52 for producing biogas from biomaterial by dry anaerobic digestion. The biogas reactor 12 has a first end 14 and a second end 16. First handling equipment 18 is set in association with the first end 14 of the biogas reactor 12 for unloading transfer containers 36. Preferably, the system also includes container handling equipment, with which the transfer container 36 is moved from a lorry 48. Alternatively, container handling equipment may be integrated into the biogas reactor; however, handling equipment for interchangeable platforms installed in lorries are preferably used as container handling equipment.

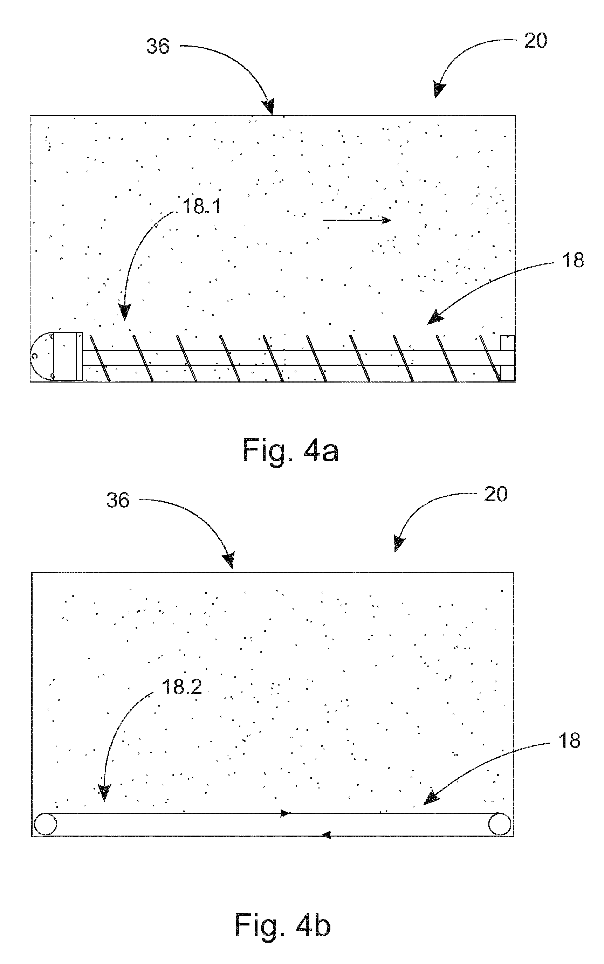

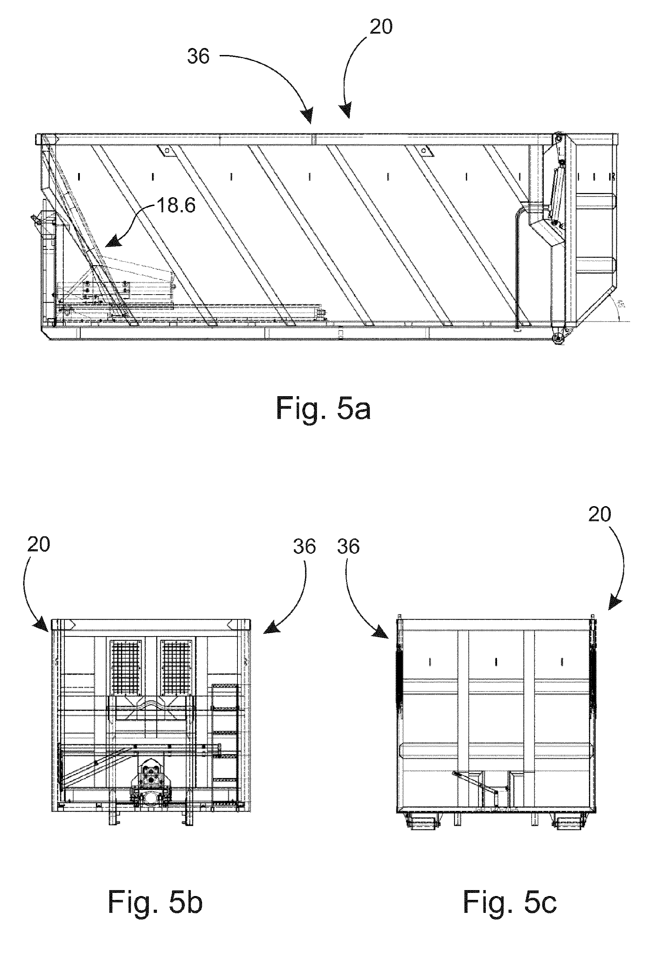

For example, the first handling equipment 18 may consist of a platform 54 according to figure 2 for supporting the transfer container 36 for unloading purposes, provided with tipping equipment for unloading biowaste from the transfer container 36. Alternatively, according to an embodiment, the first handling equipment 18 is fitted inside the transfer container 36, as in the embodiments of figures 4a to 5c. Thus, the first handling equipment 18 may consist of bottom unloading equipment according to figure 4a, for example, in which case transfer containers 36 are bottom discharging. Thus, they can be supported on the platform 54 so as to make it possible to open the unloading hatch located, for example, at one end of the bottom of the transfer container 36. The unloading hatch may also be located on the side of the transfer container. Transfer containers used can be similar to those used in the transportation of chips, silage or biowaste and they can include a pull bar unloader 18.1 functioning as the bottom discharger, located within the transfer container 36 according to figure 5a, to help unload biomaterial from the transfer container 36. As an alternative for a pull bar unloader 18.1, an unloader mat 18.2 can also be used according to figure 5b.

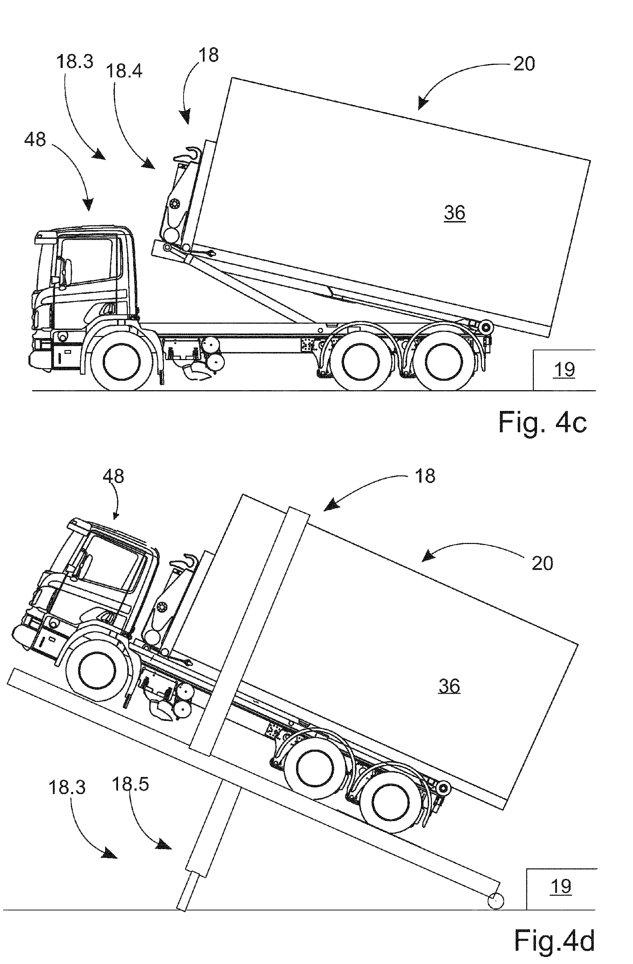

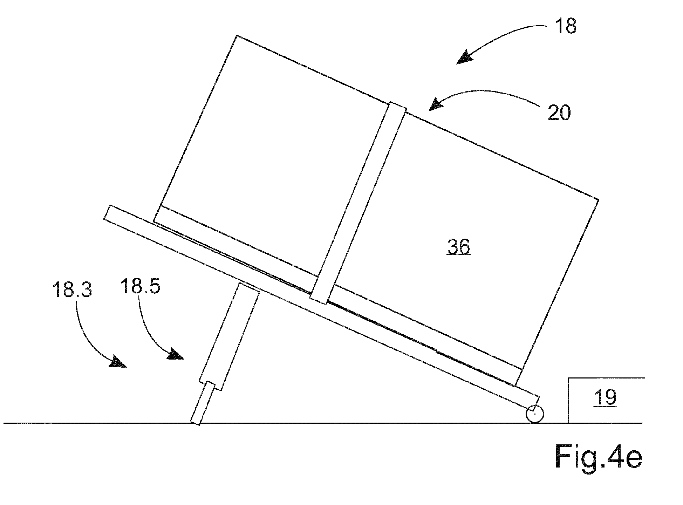

Alternatively to bottom discharge equipment, the first handling equipment 18 may consist of, for example, tipping equipment 18.3 of figure 4c, with which the transfer container 36 is tipped to unload biowaste from the transfer container 36. Tipping equipment 18.3 may consist of, according to figure 4c, either tipping equipment 18.4 integrated into a lorry 48 or, according to figure 4d, separate tipping equipment 18.5 for tipping the entire lorry. Furthermore, according to figure 4e, separate tipping equipment 18.5 can also be provided for tipping the transfer container 36 only. Furthermore, according to an embodiment, the first handling equipment 18 may consist of, according to figures 5a to 5c, a push wall 18.6 located inside the transfer container 36, which pushes the biowaste in the transfer container 36 out of the transfer container 36. Such a mechanism is very reliable and can efficiently unload the transfer container.

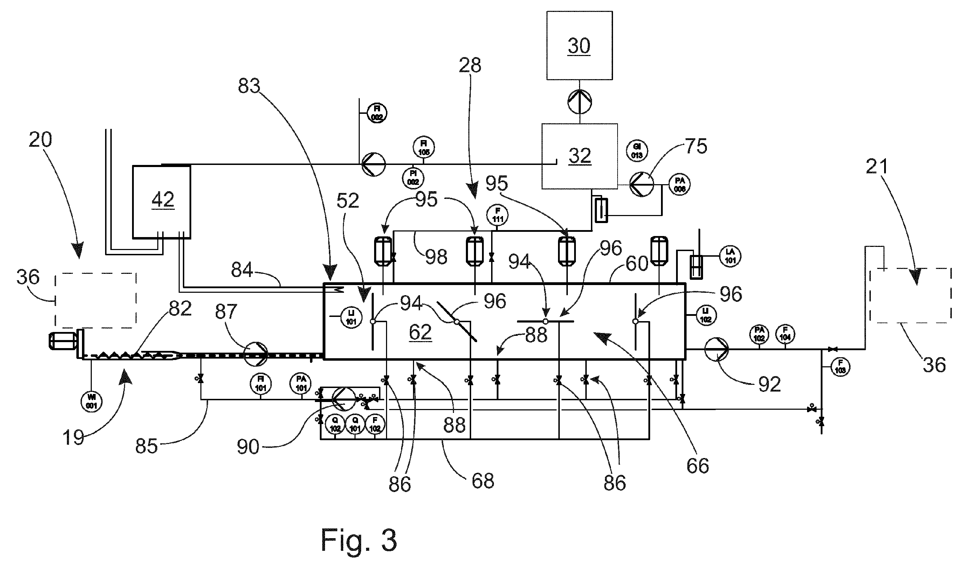

In addition to these, the system 10 includes first transfer equipment 19 set at the first end 14 of the biogas reactor 12 for supplying unloaded biomaterial to the first end 14 of the biogas reactor 12. Preferably, the first transfer equipment 19 (feed equipment) may consist of, for example, of a screw conveyor 82 according to figure 3 located below the platform 54 to transport the biomaterial dropping from the transfer container 36 from the first end 14 of the biogas reactor 12 into the biogas reactor 12. Preferably, several transfer containers are used in the system, but the system can basically also be implemented with only one transfer container.

The biogas reactor 12 of the system 10 according to the invention is preferably of a modular construction; i.e. the biogas reactor 12 consists of individual reactor blocks 52 placed successively one after another to form a channel-like structure for the biogas reactor 12. A channel-like construction enables a digestion process proceeding as a plug flow inside the biogas reactor 12. The greatest advantage of the modular construction is achieved in that the biogas reactor can be manufactured in a factory and reactor blocks are only connected together on the application site. At least the constructions related to the biogas reactor of the system and the upgrading unit are preferably placed in container-type elements, which are prefabricated in a factory. Prefabrication enables reduction of manufacturing costs of biogas reactors to a remarkably low level compared to prior art reactors. In practice, this is realised by equipping each prefabricated element with all of the additional apparatuses, instruments, wiring and piping required by the biogas reactor and the upgrading unit so that prefabricated units can be easily taken into use and combined to achieve a suitable capacity. Deviating from what is set forth above, a system according to the invention can also be implemented by assembling the biogas reactor and the upgrading unit on site, according to prior art, inside a separate building. In this case, however, the advantages of the modular construction set forth above are not achieved.

The biogas reactor used in a system according to the invention is preferably a biogas reactor 12 according to figure 3 including a frame 60, which delimits a reaction space 62 inside it for biomass, said frame 60 being channel-like and horizontal for plug flow of biomass. The frame 60 may include at least three successive reactor blocks 52 including their microbial strains and agitation equipment 66 for agitating biomass and feeding microbes to biomass and advancing biomass in the reaction space 62. Agitation equipment 66 may include a reject collection and feed system 68 for collecting reject from the reactor blocks 52 and feeding it at least to the reactor block 52 as high consistency stock.

A biogas reactor like the one set forth above can be operated using the following steps. First, the biogas reactor 12 is supplied with an input by feeding biomass to the reaction space 62 using mechanical first transfer equipment 19 while simultaneously advancing biomass in the reaction space 62 in the horizontal direction as plug flow. The first transfer equipment 19 may include, according to the figure, a feed screw 82 and a pump 87 to which biomaterial has been delivered by tipping the transfer container 36 using the first handling equipment. Biomass is agitated separately in each block in the reaction space 62 divided into successive reactor blocks 52 for feeding biomass to block-specific microbial strains and transferring biomass further in the reaction space 62, which has at least three reactor blocks 52 each with a main microbial strain of its own. The microbial strain of each reactor block 52 is preferably fed in front of the corresponding reactor block and, in at least two of these reactor blocks 52, the supply is reject obtained from the reactor block 52 as high consistency stock. Reject is preferably recirculated to the agitation equipment 96 located in the reaction space 62 with the reject recovery and recirculation system 68 via discharge connectors 88 located at the bottom of the reaction space 62 of the biogas reactor 12. The agitation equipment 96 includes feed connectors 94, through which reject can be transferred to the previous reactor block 52 as high consistency stock. Reject can also be transferred back to the front of the biogas reactor as an input with a high consistency stock pump 90 using the pipework 85. The reject recovery and recirculation system 68 is operated via valves 86 one reactor block 52 at a time. The agitation equipment 96 can be operated with motors 95 external to the reaction space 62. Finally, biogas generating as the result of anaerobic degradation of biomass is recovered. The method set forth above is one example of operating the biogas reactor.

For the recovery of biogas, the system includes biogas recovery equipment 28 located in association with the biogas reactor 12 for recovering biogas generating in the biogas reactor 12 during dry anaerobic digestion of biomaterial in a high-pressure depot 30. The recovery equipment 28 may consist of connections formed in the upper part of the reactor frame 60, through which biogas can be discharged to recovery channels. Biogases generating as a result of digestion create an overpressure in the biogas reactor, which can be depressurised via the recovery channels. Following the recovery channels, a low-pressure biogas depot may be provided from which biogas is conveyed to a compressor, which pressurises biogas to 5 - 20 bar for the upgrading unit 32.

Purified biogas discharging from the upgrading unit can be pressurised even to a higher pressure for storage purposes. Preferably, biogas is stored in a high-pressure depot 30, which is adapted to store biogas at a pressure of 200 - 300 bar. When applying such a storage pressure, the pressure of biogas is directly suitable for the refueling unit. According to figure 3, following the last reactor block 52, a discharge pump 92 may be provided as discharge equipment 22 for the system to discharge digestate with a notably lower dry matter content compared to that of the raw material to a transfer container, which is not shown in this figure. According to figure 3, the reactor space 62 is formed as a sealed space with pumps 87 and 92 wherein no oxygen is present to enable anaerobic fermentation. The biogas reactor 12 may also preferably include a liquid heating circuit 84, and the liquid contained therein can be heated in a gas boiler 42 using the combustible biogas produced. In this embodiment, the temperature control system 83 of the biogas reactor 12 is implemented with a heating circuit 84.

As mentioned previously, the system 10 also includes a biogas upgrading unit 32 placed prior to the biogas depot 30 for purifying biogas and a refueling unit 34 for distributing biogas from the depot 30 for further use. The biogas produced from biomaterial in the biogas reactor as a result of reactions contains approximately 50-75% by volume of methane (CH₄) and the rest is mainly carbon dioxide (CO₂). In addition to these, biogas may contain nitrogen and small amounts of other gases and impurities, such as 100 - 3,000 ppm of hydrogen sulphide (H₂S). Prior art includes several commercially viable technologies for upgrading biogas. Carbon dioxide removal may be based on absorption, adsorption or membrane technologies. The most common of these technologies is based on absorption of carbon dioxide in either water or a chemical. The second most common technology is based on chemical adsorption or so-called pressure swing adsorption (PSA). When using the PSA technology, zeolite, for example, can be used as the sorbent. Hydrogen sulphides can be discharged by cooling/condensing biogas and using an activated carbon filter. During cooling, raw biogas is pressurised to a level of 25 mbar, after which the temperature of gas is decreased to -25°C. Thus, the major part of hydrogen sulphides is removed from biogas with the generating condensate. A low pressure level during hydrogen sulphide discharge enables the use of more affordable and lighter constructions in the equipment meant for the hydrogen sulphide discharge. To bring the amount of impurities in gas to a level as low as possible, after this, biogas can be led to an activated carbon filter, which is preferably a catalyst. The catalyst removes remaining hydrogen sulphides almost completely. The description set forth above is only one of the possible methods for carrying out biogas purification and it should be understood that the upgrading unit in a system according to the invention can also be implemented using a different technology.

As the refueling unit 34, prior art refueling units can be used, wherein a higher pressure is preferably applied compared to that of prior art refueling units for reducing the storage volume of biogas. At the same time, biogas can be discharged from the depot to the gas container of the user's vehicle without compressing it. From the refueling unit 34, the end user can refuel the vehicle 50 with biogas.

Following the biogas reactor 12, the system 10 includes second transfer equipment 22 (discharge equipment) set at the second end 16 of the biogas reactor 12 for discharging digestate generated from biomaterial from the second end 16 of the biogas reactor 12. The second transfer equipment 22 may consist of, for example, a transfer screw or a discharge pump 92, which transfers the digestate that is periodically supplied out from the second end 16 of the biogas reactor 12 further towards a conveyor unit 20 or the transfer container 36. The discharge pump is preferably a high consistency stock pump. The dry matter content of digestate may range between 15 and 17 m-% and thus, it can be transferred with, for example, a pump from the second end of the biogas reactor from the opening on top of the transfer container to the transfer container via a feed pipe. For supplying digestate to the transfer container, the system 10 may include second handling equipment 24, which may consist of a platform 54, upon which the transfer container is supported for the duration of loading, and a pipework for leading digestate to the transfer container from the unloading discharge pump 92. In this position, the load-carrying vehicle can pick up the transfer container.

According to an embodiment, the system 10 may also include a gas boiler 42 according to figure 2 for meeting the heating demand of the system 10 using the biogas produced. The gas boiler 42 may be located in the same element 72 as the biogas upgrading unit 32, wherein an automation room 74 may additionally be provided. This makes it possible to keep the outer dimensions of the element 76 containing the biogas reactor 12 such that it is relatively easy to transport the element 76 to the application site in a complete assembly. According to figure 2, spaces may be provided at the ends of the element 76 for platforms 54 included in the first handling equipment 18 and the second handling equipment 24 of the transfer container. In addition, an equipment room 80 may be provided inside the element 76 at both ends of the biogas reactor 12 for locating equipment related to the operation of the biogas reactor 12. Furthermore, the refueling unit 34 may be a unit separate from the elements 72 and 76, which is easily accessible with a passenger car or other vehicle utilising biogas. In association with the refueling unit 34, a depot 30 is preferably included for purified biogas. According to figure 2, the production capacity of the system can be increased in a very simple way by adding a second biogas reactor beside the biogas reactor. In this case, the system must be modified in such a way that the first transfer equipment 19 and the second transfer equipment 22 feed and discharge the two biogas reactors dividing the biomaterial discharged from the transfer container into two separate material flows.

In a system according to the invention, the first handling equipment 18 and preferably the second equipment 24, too, both include vacuum generating equipment 44 to create a vacuum in the transfer container 36 for preventing odour emissions. In practice, this means that so-called lip seals may be provided in association with the platform 54 included in the handling equipment 18 and 24 to enable vacuum generation in spaces 78 and 79 shown in figure 2 remaining at the ends of the element 76. For example, sealing can be provided with a sealing system used in frozen goods terminals. With the vacuum, access of hydrogen sulphides present in the spaces 78 and 79 to the environment can be efficiently prevented and the system will not cause odour impacts to the environment. This is particularly important to ensure that the system can also be located near residential areas in the vicinity of good traffic connections, if necessary. Hydrogen sulphides aspirated with a vacuum can be ozonated for eliminating odours before releasing them to the environment.

Preferably, the system is designed in such a way that any maintenance operations will not require stopping of the process but all maintenance can be performed on the outside of the biogas reactor. A system composed of elements can be completely built on the ground, i.e. the necessary ground preparation and foundations are fairly light and the system can even be moved at moderate costs, if necessary.

A system according to the invention can be used to produce biomethane for traffic use cost-efficiently from biodegradable municipal and agricultural waste and secondary flows. The system can be located in the vicinity of traffic routes, i.e. near fuel customers, since biomaterials are brought to the plant with interchangeable platform containers, pre-processed, if necessary. The system operator may be for example a waste-treatment entrepreneur or an agricultural entrepreneur. The system is preferably continuously operating, equipped with remote monitoring and fully automated; therefore, the onsite workforce requirement for its maintenance is very low.

The reactor constructions and other necessary constructions of the system are preferably located in elements, which can be manufactured in a factory and transported to the site as complete assemblies. For example, the elements may be marine containers, which are easy to transport and affordable in terms on investments. The use of prefabricated elements in the implementation of a biogas plant enables serial manufacture and thereby lower costs per unit as well as high quality. The system can be quickly installed and taken into use on site, which reduces expensive work on site during the construction, which is one of the central cost-increasing factors in the implementation of traditional systems. In addition, the treatment capacity of the system can be easily increased by increasing the number of parallel bioreactors. If necessary, the system can even be moved at very reasonable costs. The use of elements removes the need of a separate building for the biogas reactor.

Basically, a system according to the invention is designed for the treatment of source-separated or mechanically separated biowaste; however, it also suits the treatment of other municipal and agricultural solid waste fractions (for example, plant-based biomass, dry manure, separated liquid manure, centrifuge thickened and dewatered wastewater sludge, food industry biowaste) without the addition of liquid, which enables compact reactor constructions and operating expenses by 70% lower than those of traditional systems. The biogas reactor is preferably based on a reactor construction consisting of standard-dimension reactor blocks. Since the biogas reactor is preferably composed of reactor blocks, the system is easily scalable to meet the customer's requirements and it is also easily expandable at a later stage, if necessary. If the reactor capacity needs to be later increased, additional modular elements complete with biogas reactors can preferably be connected either in parallel or in series in association with the existing reactor. Due to the compact reactor design, the surface area requirement of the system is smaller than that of traditional systems.

The output of an individual biogas reactor may range between 20 and 60 m³/h of raw gas. The treatment capacity of the system can be increased cost-efficiently by connecting biogas reactors in parallel. Correspondingly, the amount of raw material treated may range between 500 and 1000 tons/year/one biogas reactor. The total amount of treatment depends on the number of biogas reactors that are possibly used in parallel.

According to an embodiment, the outer dimensions of the transfer container may be, for example: height 2.67 m, width 2.55 m and length 7.48 m; in this case, the total volume of the interior is approximately 37 m3. Such transfer containers are manufactured for normal road traffic use by German Hadwiger Productions GmbH, for example. The dimensions of the transfer container may also vary depending on the embodiment; however, the volume of the transfer container is preferably at least 20 m³.

## Claims

1. A system for producing and distributing biogas, the system (10) including
- a biogas reactor (12) having a first end (14) and a second end (16) for producing biogas from biomaterial with dry anaerobic digestion,
- first conveyor units (20) for conveying biomaterial functioning as the raw material to the biogas reactor (12),
- first handling equipment (18) for unloading biomaterial from the first conveyor unit (20),
- first transfer equipment (19) arranged at the first end (14) of the biogas reactor (12) for supplying biomaterial to the first end (14) of the biogas reactor (12),
- second transfer equipment (22) set at the second end (16) of the biogas reactor (12) for unloading digestate formed out of biomaterial from the second end (16) of the biogas reactor (12),
- second conveyor units (21) for carrying digestate away from the biogas reactor (12),
- biogas recovery equipment (28) located in association with the biogas reactor (12) for recovering biogas produced in the biogas reactor (12) during dry anaerobic digestion of biomaterial,
- a biogas upgrading unit (32) connected to the biogas recovery equipment (28) for purifying biogas,
- a depot (30) for storing purified biogas obtained from the upgrading unit (32), and
- a refueling unit (34) for distributing biogas from the depot (30) to a user,
**characterised in that** said first conveyor units (20) and second conveyor units (21) are transfer containers (36) adapted to be transported by lorries (48), and the system (10) further includes at least one lorry (48) for transporting said transfer containers (36).

2. A system according to claim 1, **characterised in that** the biogas reactor (12) is a continuously operating plug-flow reactor.

3. A system according to claim 1 or 2, **characterised in that** said first conveyor units (20) and second conveyor units (21) are alike.

4. A system according to any of claims 1 to 3, **characterised in that** said first handling equipment (18) is formed in each first conveyor unit (20).

5. A system according to claim 4, **characterised in that** said first handling equipment (18) is a bottom conveyor fitted within a transfer container (36) that functions as said first conveyor unit (20), said bottom conveyor preferably being a pull bar unloader (18.2).

6. A system according to claim 4, **characterised in that** said first handling equipment (18) is a push wall (18.1) fitted within a transfer container (36) that functions as said first conveyor unit (20).

7. A system according to claim 4, **characterised in that** the first handling equipment (18) is floor unloading equipment fitted within a transfer container (36).

8. A system according to any of claims 1 to 3, **characterised in that** said first handling equipment (18) is tipping equipment for tipping the first conveyor unit (20).

9. A system according to any of claims 1 to 8, **characterised in that** the system (10) includes container handling equipment for moving transfer containers (36) from and to a lorry (48).

10. A system according to any of claims 1 to 9, **characterised in that** the system (10) includes second handling equipment (24) for filling transfer containers (36) with digestate discharging from the biogas reactor (12).

11. A system according to claim 10, **characterised in that** both the first handling equipment (18) and the second handling equipment (24) include vacuum-generation equipment (44) for generating a vacuum in the transfer container (36) to prevent odour emissions.

12. A system according to any of claims 1 to 11, **characterised in that** said refueling unit (34) is a refueling unit (34) for biomethane upgraded for road traffic purposes.

13. A system according to any of claims 1 to 12, **characterised in that** the first conveyor units (20) and the second conveyor units (21) are the same transfer containers (36).

14. A system according to any of claims 1 to 13, **characterised in that** the system (10) also includes a gas boiler (42) for meeting the heating demand of the system (10) using the biogas produced.

15. A system according to any of claims 1 to 14, **characterised in that** the system (10) includes automation equipment for automating the functions of the system (10).
